# EUROPEAN PATENT SPECIFICATION

(11) **EP 2 208 705 B1**
(45) Date of publication and mention of the grant of the patent: **17.06.2015**
(21) Application number: 08832423.1
(22) Date of filing: 18.09.2008
(51) Int. Cl.: C01B 25/32, A23L 1/03, A61K 9/20, A61K 9/48, A61K 47/04, A23L 1/00, A61K 47/02

(54) **CALCIUM SECONDARY PHOSPHATE ANHYDRIDE PARTICLE AND METHOD FOR PRODUCING THE SAME**
CALCIUMHYDROGENPHOSPHATANHYDRID-PARTIKEL UND HERSTELLUNGSVERFAHREN DAFÜR
PARTICULE D'ANHYDRIDE DE PHOSPHATE SECONDAIRE DE CALCIUM ET SON PROCÉDÉ DE FABRICATION

(30) Priority: 20.09.2007 JP 2007243488
(43) Date of publication of application: 21.07.2010
(73) Proprietor: KYOWA CHEMICAL INDUSTRY CO., LTD., Takamatsu-shi, Kagawa 761-0113 (JP)
(72) Inventor: KITAKADO, Kazuo, Sakaide-shi KAGAWA 762-0012 (JP)
(74) Representative: Duckworth, Timothy John
(86) International application number: PCT/JP2008/067358
(87) International publication number: WO 2009/038217

(56) References cited:
- JP-A- 59 223 207
- JP-A- 59 223 207
- JP-A- 2005 289 763
- JP-A- 2007 126 452
- JP-B1- 51 031 238
- US-A- 3 829 562
- US-A- 5 338 524
- DATABASE WPI Week 200575 Thomson Scientific, London, GB; AN 2005-729395 XP002690320, -& JP 2005 289763 A (YABASHI KOGYO KK) 20 October 2005 (2005-10-20)
- DATABASE WPI Week 197640 Thomson Scientific, London, GB; AN 1976-75026X XP002690321, -& JP 51 031238 B (KYOWA CHEM IND CO LTD) 6 September 1976 (1976-09-06)
- DATABASE WPI Week 200739 Thomson Scientific, London, GB; AN 2007-413130 XP002690322, -& WO 2007/043542 A1 (OTSUKA PHARM CO LTD) 19 April 2007 (2007-04-19)
- 'Kagaku Daijiten 7', 10 November 1979, KYORITSU SHUPPAN CO., LTD. pages 606 - 609, XP008134309

## Description

### TECHNICAL FIELD

The present invention relates to anhydrous dicalcium phosphate particles, a method of producing the same and a vehicle containing the same. That is, the present invention relates to anhydrous dicalcium phosphate particles which are preferred as a vehicle for medications and food additives, a powder, a capsule filling powder or a nucleating agent and to a production method thereof. More specifically, it relates to anhydrous dicalcium phosphate particles which are agglomerated with a small variation in size, have high capsule filling work efficiency due to their small bulk and high flowability, are also preferred as a nucleating agent for granulation, are excellent in tableting properties and provide a small tablet by a direct tableting method and to a production method thereof.

### BACKGROUND OF THE ART

Although anhydrous dicalcium phosphate particles were produced by heating a water suspension of hydrous dicalcium phosphate at 50 to 100°C, in recent years, they have also been synthesized by reacting a calcium compound such as slaked lime with phosphoric acid and processed into not only the above vehicle but also the powder or capsule filling powder to be used as a source of supplying a calcium component and a phosphoric acid component or a dentifrice. When they are used as the above powder or capsule filling powder, spherical anhydrous dicalcium phosphate particles which have a small bulk and high flowability are preferred from the viewpoint of handling properties.

JP-B 51-31238 discloses a method of obtaining anhydrous dicalcium phosphate fine powders having high tableting strength by adding an alkali when anhydrous dicalcium phosphate is obtained by heating hydrous dicalcium phosphate in a water suspension. The obtained anhydrous dicalcium phosphate fine powders have a large bulk and low flowability.

JP-A 59-223206 discloses that spherical anhydrous dicalcium phosphate is obtained by adding a phosphoric acid condensate together with lime milk when a reaction solution begins to emulsify in a known method of producing anhydrous dicalcium phosphate by adding lime milk to a phosphoric acid aqueous solution heated at 75°C to carry out a neutralization reaction. Although the obtained anhydrous dicalcium phosphate has excellent flowability and tableting properties, it has a large bulk and is unsatisfactory in terms of handling properties.

JP-A 59-223207 describes coagulated fine-grained anhydrous calcium secondary phosphate and its manufacture.

JP-A 59-223208 discloses that agglomerated anhydrous dicalcium phosphate is obtained by adding an electrolyte together with lime milk when a reaction solution begins to emulsify in a known method of producing anhydrous dicalcium phosphate by adding lime milk to a phosphoric acid aqueous solution heated at 75°C to carry out a neutralization reaction. The obtained agglomerated anhydrous dicalcium phosphate is a dentifrice, and this reference does not mention the tableting properties of a solid preparation at all. A salt of an alkali metal, an alkali earth metal or an aluminum-based metal and phosphoric acid, sulfuric acid, hydrochloric acid or nitric acid is given as the electrolyte added in the above production method. However, according to the knowledge of the inventor of the present invention, an agglomerate having a small bulk, excellent tableting properties and high flowability cannot be obtained from the above salt.

As described above, the anhydrous dicalcium phosphate of the prior art is unsatisfactory in terms of handling and tableting properties.

### DISCLOSURE OF THE INVENTION

It is an object of the present invention to provide anhydrous dicalcium phosphate particles whose agglomerates have a small variation in size and which have a small bulk, high tableting strength, excellent flowability and excellent handling properties while the dispersibility of the agglomerate is enhanced.

It is another object of the present invention to provide anhydrous dicalcium phosphate particles which satisfy the conflicting requirements of excellent flowability, a small bulk and excellent tableting properties at the same time during preparation and a production method thereof.

In the direct tableting method, anhydrous dicalcium phosphate particles which have excellent handling properties and tableting properties and provide a small tablet are required as a vehicle. In the present invention, "excellent tableting properties" means that tableting strength is high and capping and chipping hardly occur and "excellent handling properties" means that flowability is high and the bulk is small. When one of these requirements is missing, handling properties deteriorate.

The inventors of the present invention have found that anhydrous dicalcium phosphate particles whose agglomerates have a small variation in size and which have a small bulk, high tableting strength and excellent flowability and handling properties while the dispersibility of the agglomerates is enhanced can be attained by setting the average particle diameter of the agglomerates measured by a laser diffraction scattering method to 15 to 70 µm, the variation coefficient for the average particle diameter to not more than 55 % and the apparent volume weight ratio to 0.5 to 1.5 ml/g. The preferred lower limit of the above average particle diameter is 20 µm and the preferred upper limit is 50 µm. The preferred lower limit of the above apparent volume weight ratio is 0.7 ml/g and the preferred upper limit is 1.3 ml/g.

The inventors of the present invention have found that agglomerated anhydrous dicalcium phosphate particles of interest are obtained by heating a phosphoric acid compound and a calcium compound at 75°C or higher in advance to carry out a neutralization reaction in the presence of an alkali. As a matter of course, the alkali does not contain a calcium compound which involved in the reaction. Therefore, to carry out the neutralization reaction in the presence of the alkali in the present invention means that an alkali except for the calcium compound is existent in the reaction.

As understood from the disclosure of the above JP-B 51-31238, since the anhydrous dicalcium phosphate particles produced in the presence of an alkali have a large bulk and low flowability, it has been considered that the object of the present invention cannot be attained. The inventors of the present invention have obtained an utterly unexpected result that agglomerated anhydrous dicalcium phosphate particles having a small bulk and high flowability are obtained by combining the addition of an alkali with the method in which a phosphoric acid compound and a calcium compound are heated at 75°C or higher in advance to carry out a neutralization reaction.

The method of producing anhydrous dicalcium phosphate particles of the present invention will be described in more detail hereinbelow.

The anhydrous dicalcium phosphate particles of the present invention are obtained by heating water suspensions of a phosphoric acid compound and a calcium compound at 75 to 100°C in advance to carry out a neutralization reaction. The preferred temperature is 75 to 85°C. When the temperature is lower than 70°C, a hydrate may be formed and when the temperature is higher than 100°C, the dramatic increase of the effect cannot be expected and it is not economical. The reaction temperature may be maintained at 75 to 105°C when it is taken into consideration that the reaction between the calcium compound and the phosphoric acid compound is an exothermal reaction. However, when the ratio of an anhydride to the reaction product is taken in account, it is more preferred to control the reaction temperature to a range of 90 to 105°C.

The reaction is carried out by injecting the phosphoric acid compound into a reactor, heating and stirring it and adding the calcium compound to it. After the reaction, washing, dehydration and drying are carried out.

The molar ratio of the calcium compound to the phosphoric acid compound in the above neutralization reaction is 0.8 to 1.0, preferably 0.9.

Anhydrous dicalcium phosphate particles formed by adding the alkali during the neutralization reaction are agglomerated and have improved flowability with an apparent volume weight ratio of not more than 1.5 ml/g. To set the average particle diameter measured by the laser diffraction scattering method to 15 to 70 µm and the variation coefficient of the particles for the average particle diameter to not more than 50 %, the addition of the alkali is started when the molar ratio of the calcium compound to the phosphoric acid compound (molar ratio of [CaO]/[H₃PO₄]) becomes 0.2 to 0.5 after the addition of the calcium compound is started. When the phosphoric acid compound is an acidic calcium phosphate solution, the above [CaO] includes calcium contained in the acidic calcium phosphate. If the addition of the alkali is started when the above molar ratio is less than 0.2, a variation in size of the agglomerates becomes so large that the variation coefficient becomes not less than 50 %, resulting in the low flowability of the product. If the addition is started when the molar ratio exceeds 0.5, the flowability of the product degrades and the apparent volume weight ratio becomes not less than 1.5 ml/g.

The amount of the alkali added is adjusted to ensure that the molar ratio of the alkali to the phosphoric acid compound (molar ratio of [alkali] to [phosphoric acid compound]) becomes 0.015 to 0.35. It is preferably 0.034 to 0.17. When the molar ratio is lower than 0.015, the flowability of the product degrades and the bulk becomes large. When the molar ratio exceeds 0.35, unreacted lime remains in the product.

The phosphoric acid compound used in the present invention is selected from phosphoric acid, ammonium phosphate and alkali metal salts of phosphoric acid and the like. Specific examples of the phosphoric acid compound include phosphoric acid, sodium phosphate, potassium phosphate and an acidic calcium phosphate solution. Out of these, phosphoric acid or an acidic calcium phosphate solution is preferred, and phosphoric acid is most preferred. They may be used as an aqueous solution.

The calcium compound used in the present invention is selected from calcium oxide (quicklime), calcium hydroxide (slaked lime) and calcium chloride and the like. Out of these, calcium hydroxide is most preferred because it can be used as lime milk when it is dispersed in water before use.

The alkali which is added for the neutralization reaction in the present invention is selected from sodium hydroxide, potassium hydroxide and ammonia water and the like. Out of these, sodium hydroxide and potassium hydroxide are preferred.

According to the present invention, there are provided the following agglomerated anhydrous dicalcium phosphate particles and the following method of producing anhydrous dicalcium phosphate particles.
(1) Agglomerated anhydrous dicalcium phosphate particles which satisfy both of the following requirements (a) and (b) :
   (a) an average particle diameter measured by a laser diffraction scattering method of 15 to 70 µm and a variation coefficient of the particles for the average particle diameter of not more than 55 %; and
   (b) an apparent volume weight ratio of 0.5 to 1.5 ml/g.
(2) The above agglomerated anhydrous dicalcium phosphate particles (1) which have an average particle diameter measured by the laser diffraction scattering method of 20 to 50 µm and a variation coefficient of the particles for the average particle diameter of 30 to 55 %.
(3) The above agglomerated anhydrous dicalcium phosphate particles (1) which have an apparent volume weight ratio of 0.7 to 1.3 ml/g.
(4) A method of producing the above agglomerated anhydrous dicalcium phosphate particles (1), (2) or (3) by heating a water suspension of dicalcium phosphate dihydrate formed from a neutralization reaction between a phosphoric acid compound and a calcium compound in the presence of an alkali additive, which method comprises heating the phosphoric acid compound and the calcium compound at 75 to 100 °C respectively in advance, thereafter starting the addition of the calcium compound to the phosphoric acid compound, and thereafter starting the addition of the alkali to the reaction solution when the molar ratio of the calcium compound to the phosphoric acid compound becomes 0.2 to 0.5, and wherein the amount of the alkali is a value which ensures that the molar ratio of the alkali to the phosphoric acid compound becomes 0.015 to 0.35.
(5) The method (4) of producing the agglomerated anhydrous dicalcium phosphate particles (1) to (3), wherein the phosphoric acid compound is phosphoric acid.
(6) The method (4) of producing the agglomerated anhydrous dicalcium phosphate particles (1) to (3), wherein the calcium compound is quicklime or slaked lime.
(7) The method (4) of producing the agglomerated anhydrous dicalcium phosphate particles (1) to (3), wherein the alkali is sodium hydroxide.
(8) A pharmaceutical vehicle which contains the agglomerated anhydrous dicalcium phosphate particles (1) to (3).
(9) A pharmaceutical powder which contains the agglomerated anhydrous dicalcium phosphate particles (1) to (3).
(10) A pharmaceutical capsule which contains the agglomerated anhydrous dicalcium phosphate particles (1) to (3).
(11) A pharmaceutical nucleating agent which contains the agglomerated anhydrous dicalcium phosphate particles (1) to (3).
(12) A vehicle for food additives which contains the agglomerated anhydrous dicalcium phosphate particles (1) to (3).

In the above preparations (8) to (12), the agglomerated anhydrous dicalcium phosphate particles are preferably contained in an amount of 1 to 95 wt%. Further, in the pharmaceutical preparations (9) and (10), the total amount of an effective component (main agent), a vehicle containing agglomerated anhydrous dicalcium phosphate particles and a lubricant is preferably 85 to 97 wt%.

According to the above production method, agglomerated anhydrous dicalcium phosphate particles having a low content of a heavy metal such as arsenic are obtained.

### BRIEF DESCRIPTION OF THE DRAWINGS

Fig. 1 is an X-ray diffraction diagram of the agglomerated anhydrous dicalcium phosphate particles of the present invention obtained in Synthesis Example 1 of Example 1;
Fig. 2 is an X-ray diffraction diagram of dicalcium phosphate particles described in JCPDS; and
Fig. 3 is an electron microphotograph of the agglomerated anhydrous dicalcium phosphate particles of the present invention obtained in Synthesis Example 1 of Example 1.

### BEST MODE FOR CARRYING OUT THE INVENTION

The following examples are given to further illustrate the present invention.

In the examples, (a) the average particle diameter measured by the laser diffraction scattering method and the variation coefficient of the particles and (b) the apparent volume weight ratio of the anhydrous dicalcium phosphate of the present invention are values measured by the following methods.

### (a) average particle diameter and variation coefficient of particles

The average particle diameter (mv) and the standard deviation (sd) of the particle diameter were measured with MICROTRAC Particle Size Analyzer SPA type MT3300 (of Leeds & Northrup Instruments) in accordance with the following method, and the variation coefficient was calculated from these measurement results.

After 700 mg of a powder sample was added to 70 ml of an aqueous solution containing 0.2 wt% of sodium hexametaphosphate and dispersed in the solution with ultrasonic waves (Model US-300 of NISSEI Corporation., current of 300 µA) for 3 minutes, 2 to 4 ml of the obtained dispersion was collected while it was stirred with a stirrer and added to the sample chamber of the above particle size analyzer containing 250 ml of deaerated water, and the analyzer was activated to circulate the suspension for 3 minutes so as to measure the particle size distribution. This measurement was made two times in total, and "sd" and "mv" obtained by the measurement were taken as the standard deviation and the average particle diameter of the sample. Variation coefficient (%) = standard deviation of particle diameter (sd)/average particle diameter (mv) x 100

### (b) apparent volume weight ratio

This was measured in accordance with JIS K 5101.

### (c) repose angle

This was measured with the FK type repose angle measuring instrument (of Konishi MFG Co., Ltd.). Measurement conditions:
dropping height: 10 cm
Strength of vibrator: strong (50 graduations)
Outlet width of scooping board: 1.0 cm
Slit scale: 10

### (d) arsenic

This was measured by an atomic absorption method.

### Example 1

### Synthesis Example 1

2.6 liters of lime milk having a CaO content of 80 g/L and 1 liter of a phosphoric acid aqueous solution having a concentration of 405 g/L were prepared by heating at 85°C. The phosphoric acid aqueous solution heated at 85°C was injected into a reactor and the lime milk heated at 85°C was added to the aqueous solution under agitation. After the molar ratio of CaO to H₃PO₄ reached 0.3, the injection of a sodium hydroxide aqueous solution into the reactor was started. The total amount of the sodium hydroxide aqueous solution added was adjusted to ensure that the molar ratio of Na to P became 0.015. The temperature of the reaction solution was maintained at 85°C or higher. After the end of a reaction, the obtained slurry was washed and dried at 120°C for 2 hours to obtain agglomerated anhydrous dicalcium phosphate particles. The reaction conditions and the measured physical property values are shown in Table 1.

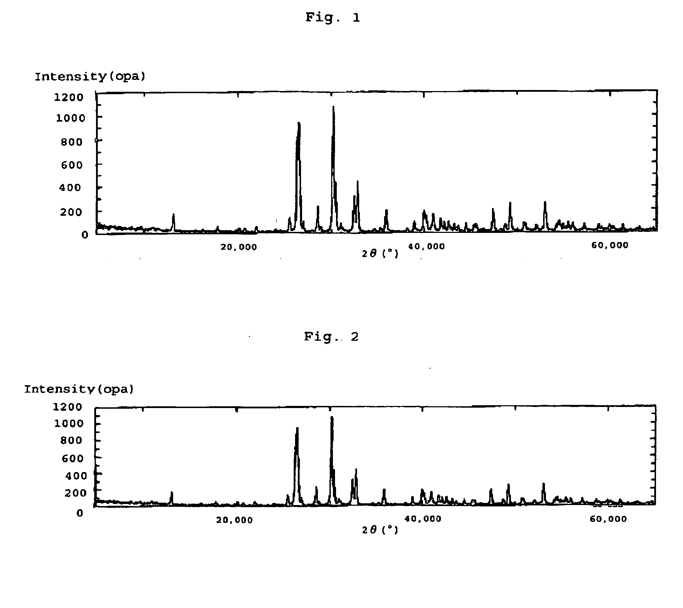

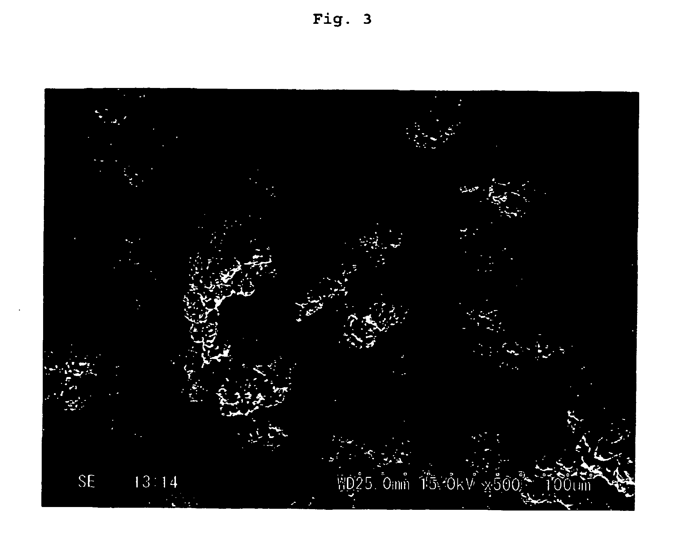

Fig. 1 and Fig. 2 are an X-ray diffraction diagram of the obtained agglomerated anhydrous dicalcium phosphate particles of the present invention and an X-ray diffraction diagram of dicalcium phosphate particles described in JCPDS as a comparison, respectively. Fig. 3 is an electron microphotograph of the above agglomerated anhydrous dicalcium phosphate particles. "JCPDS" stands for the Joint Committee of Powder Diffraction Standards and represents a standard XRD database of powder samples.

### Synthesis Example 2 (Comparative Example)

When a reaction was carried out in the same manner as in Synthesis Example 1 except that the total amount of the sodium hydroxide aqueous solution added was adjusted to ensure that the molar ratio of Na to P became 0.010, agglomerated anhydrous dicalcium phosphate particles were obtained. The reaction conditions and the measured physical property values are shown in Table 1.

### Synthesis Example 3 (Comparative Example)

When a reaction was carried out in the same manner as in Synthesis Example 1 except that the total amount of the sodium hydroxide aqueous solution added was adjusted to ensure that the molar ratio of Na to P became 0.42, a mixture of agglomerated anhydrous dicalcium phosphate particles and powdery calcium hydroxide particles was obtained. The reaction conditions and the measured physical property values are shown in Table 1.

### Synthesis Example 4 (Comparative Example)

2.6 liters of lime milk having a CaO content of 80 g/L and 1 liter of a phosphoric acid aqueous solution having a concentration of 405 g/L were heated at 50°C. The phosphoric acid aqueous solution heated at 50°C was injected into a reactor and the lime milk heated at 50°C was added to the aqueous solution under agitation. After the molar ratio of CaO to H₃PO₄ reached 0.3, the injection of a sodium hydroxide aqueous solution into the reactor was started. The total amount of the sodium hydroxide aqueous solution added was adjusted to ensure that the molar ratio of Na to P became 0.015. When the obtained slurry was washed and dried after the end of a reaction, a mixture of anhydrous dicalcium phosphate and dicalcium phosphate·dihydrate was obtained. The reaction conditions and the measured physical property values are shown in Table 1.

### Synthesis Example 5 (Comparative Example)

When a reaction was carried out in the same manner as in Synthesis Example 1 except that pyrophosphoric acid was injected in place of the sodium hydroxide aqueous solution to ensure that the weight ratio of pyrophosphoric acid to CaO became 20 %, spherical anhydrous dicalcium phosphate particles were obtained. The reaction conditions and the measured physical property values are shown in Table 1.

### Synthesis Example 6

1.16 L of lime milk having a CaO content of 80 g/L and an acidic calcium phosphate solution (calcium dihydrogen phosphate monohydrate to be added to food having a concentration of 521 g/2.44 L) were heated at 100°C. The acidic calcium phosphate solution heated at 100°C was injected into a reactor and the lime milk heated at 100°C was added to the solution under agitation. The injection of a sodium hydroxide aqueous solution was started when the molar ratio of CaO to H₃PO₄ became 0.5 ([CaO] includes the amount of CaO contained in the acidic calcium phosphate). The total amount of the sodium hydroxide aqueous solution added was adjusted to ensure that the molar ratio of Na to P became 0.17. The temperature of the reaction solution was maintained at 100°C or higher. When the obtained slurry was washed and dried after the end of a reaction, agglomerated anhydrous dicalcium phosphate particles were obtained. The reaction conditions and the measured physical property values are shown in Table 1.

### Synthesis Example 7

2.6 L of lime milk having a CaO content of 80 g/L and 1 liter of a phosphoric acid aqueous solution having a concentration of 405 g/L were heated at 75°C. The phosphoric acid aqueous solution heated at 75°C was injected into a reactor and the lime milk heated at 75°C was added to the aqueous solution under agitation. When the molar ratio of CaO to H₃PO₄ reached 0.3, the injection of a potassium hydroxide aqueous solution into the reactor was started. The total amount of the potassium hydroxide aqueous solution added was adjusted to ensure that the molar ratio of K to P became 0.35. The temperature of the reaction solution was maintained at 85°C or higher. When the obtained slurry was washed and dried after the end of a reaction, agglomerated anhydrous dicalcium phosphate particles were obtained. The reaction conditions and the measured physical property values are shown in Table 1.

### Synthesis Example 8 (Comparative Example)

When a reaction was carried out in the same manner as in Synthesis Example 1 except that the injection of the sodium hydroxide aqueous solution into the reactor was started after the molar ratio of CaO to H₃PO₄ reached 0.15 and the total amount of the sodium hydroxide aqueous solution added was adjusted to ensure that the molar ratio of Na to P became 0.086, agglomerated anhydrous dicalcium phosphate particles were obtained. The reaction conditions and the measured physical property values are shown in Table 1.

### Synthesis Example 9 (Comparative Example)

When a reaction was carried out in the same manner as in Synthesis Example 1 except that the injection of the sodium hydroxide aqueous solution into the reactor was started after the molar ratio of CaO to H₃PO₄ reached 0.53 and the total amount of the sodium hydroxide aqueous solution added was adjusted to ensure that the molar ratio of Na to P became 0.086, agglomerated anhydrous dicalcium phosphate particles were obtained. The reaction conditions and the measured physical property values are shown in Table 1.

In Synthesis Example 4, a hydrate was formed because water suspensions of the phosphoric acid compound and the calcium compound were not heated at an appropriate temperature in advance. In Synthesis Examples 8 and 9, as the time of starting the injection of the alkali was not within the preferred range disclosed by the present invention, the product had low flowability and a large bulk. In Synthesis Example 2, as the amount of the alkali added was much smaller than the preferred range of the present invention, the product had low flowability and a large bulk. In Synthesis Example 3, as the amount of the alkali added was much larger than the preferred range of the present invention, unreacted lime remained in the product.

Since the agglomerated anhydrous dicalcium phosphate particles produced by the method of the present invention as described above have a small bulk and high flowability, they are excellent in metering and handling properties. It is best to directly use it as a powder.

**Table 1**

| | | | Synthesis Example 1 | Synthesis Example 2* | Synthesis Example 3* | Synthesis Example 4* | Synthesis Example 5* |
|---|---|---|---|---|---|---|---|
| Phosphoric acid compound | | Type | Phosphoric acid | Phosphoric acid | Phosphoric acid | Phosphoric acid | Phosphoric acid |
| | | Temperature | 85°C | 85°C | 85°C | 50°C | 85°C |
| Calcium compound | | Type | Lime milk | Lime milk | Lime milk | Lime milk | Lime milk |
| | | Temperature | 85°C | 85°C | 85°C | 50°C | 85°C |
| Type of alkali | | | Sodium hydroxide | Sodium hydroxide | Sodium hydroxide | Sodium hydroxide | Pyrophosphoric acid |
| Molar ratio of CaO to H₃PO₄ at the start of adding alkali [CaO]/[H₃PO₄] | | | 0.3 | 0.3 | 0.3 | 0.3 | - |
| Amount of alkali (molar ratio of Na to P) | | | 0.015 | 0.010 | 0.42 | 0.015 | - |
| Reaction temperature | | | 95°C | 95°C | 95°C | 65°C | 95°C |
| Reaction product | | | Anhydrous dicalcium phosphate | Anhydrous dicalcium phosphate | Anhydrous dicalcium phosphate/ Calcium hydroxide (mixture) | Dicalcium phosphate· dihydrate/ Anhydrous dicalcium phosphate (mixture) | Anhydrous dicalcium phosphate |
| Dried product | Type | | Anhydrous dicalcium phosphate | Anhydrous dicalcium phosphate | Anhydrous dicalcium phosphate/ Calcium hydroxide (mixture) | Dicalcium phosphate· dihydrate/ Anhydrous dicalcium phosphate (mixture) | Anhydrous dicalcium phosphate |
| | Apparent volume weight ratio ml/g | | 1.21 | 1.44 | 1.35 | 1.80 | 1.75 |
| | Repose angle | | 35 | 42 | 45 | 41 | 38 |
| | Average secondary particle diameter µm | | 36.6 | 60 | 31 | 18 | 25 |
| | Particle size variation coefficient % | | 40 | 71 | 76 | 70 | - |
| | Content of arsenic ppm | | 0.1 | - | - | - | - |
| | Particle shape | | Agglomerate | Agglomerate | Agglomerate/ fine powder (mixture) | Agglomerate | Spherical |

| | | | Synthesis Example 6 | Synthesis Example 7 | Synthesis Example 8* | Synthesis Example 9* | |
|---|---|---|---|---|---|---|---|
| Phosphoric acid compound | | Type | Acidic calcium phosphate solution | Phosphoric acid | Phosphoric acid | Phosphoric acid | |
| | | Temperature | 100°C | 75°C | 85°C | 85°C | |
| Calcium compound | | Type | Lime milk | Lime milk | Lime milk | Lime milk | |
| | | Temperature | 100°C | 75°C | 85°C | 85°C | |
| Type of alkali | | | Sodium hydroxide | Potassium hydroxide | Sodium hydroxide | Sodium hydroxide | |
| Molar ratio of CaO to H₃PO₄ at the start of adding alkali [CaO] / [H₃PO₄] | | | 0.5 | 0.3 | 0.15 | 0.53 | |
| Amount of alkali (molar ratio of Na to P) | | | 0.17 | 0.35 ([K] / [P]) | 0.086 | 0.086 | |
| Reaction temperature | | | 103°C | 85°C | 95°C | 95°C | |
| Reaction product | | | Anhydrous dicalcium phosphate | Anhydrous dicalcium phosphate | Anhydrous dicalcium phosphate | Anhydrous dicalcium phosphate | |
| Dried product | Type | | Anhydrous dicalcium phosphate | Anhydrous dicalcium phosphate | Anhydrous dicalcium phosphate | Anhydrous dicalcium phosphate | |
| | Apparent volume weight ratio ml/g | | 1.16 | 1.32 | 1.52 | 1.65 | |
| | Repose angle ° | | 37 | 35 | 45 | 43 | |
| | Average secondary particle diameter µm | | 63 | 21 | 13 | 31 | |
| | Particle size variation coefficient % | | 50 | 34 | 63 | 68 | |
| | Content of arsenic ppm | | 0.1 | 0.1 | - | - | |
| | Particle shape | | Agglomerate | Agglomerate | Agglomerate | Agglomerate | |

| | | | | | | | |
|---|---|---|---|---|---|---|---|
| * Comparative Example | | | | | | | |

### Example 2

The results obtained when the anhydrous dicalcium phosphate particles obtained in the above Synthesis Examples of Example 1 were tableted by means of a static compressor are shown in Table 2. The diameter of a pounder was 10 mm, the weight of each tablet was 500 mg, and the tableting pressure was 1 t.

As understood from the tableting results of Example 2, the agglomerated anhydrous dicalcium phosphate particles of the present invention are suitable for use as a vehicle because each particle is thin and obtains high tablet hardness.

**Table 2**

| | Tablet Example 1 | Tablet Example 2* | Tablet Example 3* | Tablet Example 4 | Tablet Example 5 |
|---|---|---|---|---|---|
| Powder | Synthesis Example 1 | Synthesis Example 4 | Synthesis Example 5 | Synthesis Example 6 | Synthesis Example 7 |
| Tablet hardness kg | 8.4 | 6.0 | 8.2 | 8.4 | 7.7 |
| Tablet thickness mm | 3.2 | 4.0 | 4.0 | 3.3 | 3.4 |

| | | | | | |
|---|---|---|---|---|---|
| * Comparative Example | | | | | |

### Example 3

Each of the anhydrous dicalcium phosphate particles obtained in the Synthesis Examples of Example 1 was filled into a capsule to check the adhesion of the powders (particles) to the surface of the capsule. The particles were filled into the capsule by means of a capsule filling machine (trade name: c401, manufactured by Walden Inc.) and a damper (trade name: c402, manufactured by Walden Inc.), and a gelatin No. 1 capsule (manufactured by Walden Inc.) was used. The total amount of the powders set in a holder was 50 g.

× means that the powders (particles) adhere to the surface of the capsule, ○ means that the powders (particles) slightly adhere to the surface of the capsule, and ⊚ means that the powders (particles) do not adhere to the surface of the capsule.

When the filling example 1 is compared with the filling examples 2 to 5, the agglomerated anhydrous dicalcium phosphate of the present invention does not adhere to the wall of the capsule and increases the production efficiency of capsules. Therefore, it is suitable for use as a capsule filler.

**Table 3**

| | Filling Example 1 | Filling Example 2* | Filling Example 3* | Filling Example 4 | Filling Example 5 |
|---|---|---|---|---|---|
| Powder | Synthesis Example 1 | Synthesis Example 4 | Synthesis Example 5 | Synthesis Example 6 | Synthesis Example 7 |
| Adhesion of powders to the outer surface of capsule | ⊚ | × | ○ | ○ | ○ |

| | | | | | |
|---|---|---|---|---|---|
| * Comparative Example | | | | | |

### Industrial Applicability

By the production method of the present invention, there can be provided an agglomerated anhydrous dicalcium phosphate particle vehicle which has excellent handling properties and is advantageous to obtain a small-sized tablet by a direct tableting method. Further, there can be provided agglomerated anhydrous dicalcium phosphate particles which are excellent in metering and handling properties as they have a small bulk and high flowability and suitable for use as a powder or a capsule filler.

## Claims

1. Agglomerated anhydrous dicalcium phosphate particles which satisfy both of the following requirements (a) and (b):
(a) an average particle diameter measured by a laser diffraction scattering method of 15 to 70 µm and a variation coefficient of the particles for the average particle diameter of not more than 55 %; and
(b) an apparent volume weight ratio of 0.5 to 1.5 ml/g.

2. The agglomerated anhydrous dicalcium phosphate particles according to claim 1 which have an average particle diameter measured by the laser diffraction scattering method of 20 to 50 µm and a variation coefficient of the particles for the average particle diameter of 30 to 55 %.

3. The agglomerated anhydrous dicalcium phosphate particles according to claim 1 which have an apparent volume weight ratio of 0.7 to 1.3 ml/g.

4. A method of producing the agglomerated anhydrous dicalcium phosphate particles of claim 1, 2 or 3 by heating a water suspension of dicalcium phosphate formed from a neutralization reaction between a phosphoric acid compound and a calcium compound in the presence of an alkali additive, which method comprises:
heating the phosphoric acid compound and the calcium compound at 75 to 100°C respectively in advance,
thereafter starting the addition of the calcium compound to the phosphoric acid compound, and
thereafter starting the addition of the alkali to the reaction solution when the molar ratio of the calcium compound to the phosphoric acid compound becomes 0.2 to 0.5,
wherein the amount of the alkali is a value which ensures that the molar ratio of the alkali to the phosphoric acid compound becomes 0.015 to 0.35.

5. The method according to claim 4, wherein the phosphoric acid compound is phosphoric acid.

6. The method according to claim 4, wherein the calcium compound is quicklime or slaked lime.

7. The method according to claim 4, wherein the alkali is sodium hydroxide.

8. A product which contains the agglomerated anhydrous dicalcium phosphate particles of any one of claims 1, 2 or 3, which product is a pharmaceutical vehicle, powder, capsule or nucleating agent, or a vehicle for food additives.

9. A product according to claim 8 which is a pharmaceutical vehicle.

10. A product according to claim 8 which is a pharmaceutical powder.

11. A product according to claim 8 which is a pharmaceutical capsule.

12. A product according to claim 8 which is a pharmaceutical nucleating agent.

13. A product according to claim 8 which is a vehicle for food additives.

## Patentansprüche

1. Agglomerierte wasserfreie Dicalciumphosphat-Teilchen, die die nachstehenden Bedingungen (a) und (b) erfüllen:
(a) ein durchschnittlicher Teilchendurchmesser, gemessen durch ein Laserbeugungs-Streuungsverfahren, von 15 bis 70 µm und ein Variationskoeffizient der Teilchen in Bezug auf den Teilchendurchmesser von nicht mehr als 55 %; und
(b) ein scheinbares Volumen-Gewichts-Verhältnis von 0,5 bis 1,5 ml/g.

2. Agglomerierte wasserfreie Dicalciumphosphat-Teilchen nach Anspruch 1, die einen durchschnittlichen Teilchendurchmesser, gemessen durch ein Laserbeugungs-Streuungsverfahren, von 20 bis 50 µm, und einen Variationskoeffizienten der Teilchen in Bezug auf den Teilchendurchmesser von 30 bis 55 % aufweisen.

3. Agglomerierte wasserfreie Dicalciumphosphat-Teilchen nach Anspruch 1, die ein scheinbares Volumen-Gewichts-Verhältnis von 0,7 bis 1,3 ml/g aufweisen.

4. Verfahren zur Herstellung der agglomerierten wasserfreien Dicalciumphosphat-Teilchen nach Anspruch 1, 2 oder 3 durch Erwärmen einer wässrigen Suspension von Dicalciumphosphat, das durch eine Neutralisationsreaktion zwischen einer Phosphorsäureverbindung und einer Calciumverbindung in Gegenwart eines Alkaliadditivs gebildet worden ist, wobei das Verfahren Folgendes umfasst:
vorheriges Erwärmen der Phosphorsäureverbindung bzw. der Calciumverbindung auf 75 bis 100 °C,
anschließendes Beginnen der Zugabe der Calciumverbindung zur Phosphorsäureverbindung und
anschließendes Beginnen der Zugabe von Alkali zur Reaktionslösung, wenn das Molverhältnis der Calciumverbindung zur Phosphorsäureverbindung einen Wert von 0,2 bis 0,5 erreicht hat,
wobei die Alkalimenge einem Wert entspricht, der gewährleistet, dass das Molverhältnis von Alkali zur Phosphorsäureverbindung 0,015 bis 0,35 beträgt.

5. Verfahren nach Anspruch 4, wobei es sich bei der Phosphorsäureverbindung um Phosphorsäure handelt.

6. Verfahren nach Anspruch 4, wobei es sich bei der Calciumverbindung um gebrannten Kalk oder gelöschten Kalk handelt.

7. Verfahren nach Anspruch 4, wobei es sich beim Alkali um Natriumhydroxid handelt.

8. Produkt, das die agglomerierten wasserfreien Dicalciumphosphat-Teilchen nach einem der Ansprüche 1, 2 oder 3 enthält, wobei es sich beim Produkt um einen pharmazeutischen Träger, ein pharmazeutisches Pulver, eine pharmazeutische Kapsel oder ein pharmazeutisches Nukleierungsmittel oder um einen Träger für Nahrungsmitteladditive handelt.

9. Produkt nach Anspruch 8, bei dem es sich um einen pharmazeutischen Träger handelt.

10. Produkt nach Anspruch 8, bei dem es sich um ein pharmazeutisches Pulver handelt.

11. Produkt nach Anspruch 8, bei dem es sich um eine pharmazeutische Kapsel handelt.

12. Produkt nach Anspruch 8, bei dem es sich um ein pharmazeutisches Nukleierungsmittel handelt.

13. Produkt nach Anspruch 8, bei dem es sich um einen Träger für Nahrungsmitteladditive handelt.

## Revendications

1. Particules agglomérées de phosphate dicalcique anhydre qui satisfont aux deux exigences (a) et (b) suivantes :
(a) un diamètre moyen de particules, mesuré par un procédé de diffraction laser, de 15 à 70 µm, et un coefficient de variation du diamètre moyen des particules ne dépassant pas 55 % ; et
(b) une masse volumique apparente de 0,5 à 1,5 ml/g.

2. Particules agglomérées de phosphate dicalcique anhydre selon la revendication 1, qui ont un diamètre moyen de particules, mesuré par un procédé de dispersion par diffraction de laser, de 20 à 50 µm, et un coefficient de variation du diamètre moyen des particules de 30 à 55 %.

3. Particules agglomérées de phosphate dicalcique anhydre selon la revendication 1, qui ont une masse volumique apparente de 0,7 à 1,3 ml/g.

4. Procédé pour produire les particules agglomérées de phosphate dicalcique anhydre selon la revendication 1, 2 ou 3, par chauffage d'une suspension aqueuse de phosphate dicalcique formé à partir d'une réaction de neutralisation entre un composé d'acide phosphorique et un composé du calcium en présence d'un additif alcalin, lequel procédé comprend :
chauffer à l'avance le composé d'acide phosphorique et le composé du calcium à une température de 75 à 100°C,
ensuite démarrer l'addition du composé du calcium au composé d'acide phosphorique, et
ensuite démarrer l'addition de l'additif alcalin à la solution réactionnelle quand le rapport molaire du composé du calcium au composé d'acide phosphorique devient de 0,2 à 0,5,
dans lequel la quantité de l'additif alcalin est une valeur qui assure que le rapport molaire de l'additif alcalin au composé d'acide phosphorique devienne de 0,015 à 0,35.

5. Procédé selon la revendication 4, dans lequel le composé d'acide phosphorique est l'acide phosphorique.

6. Procédé selon la revendication 4, dans lequel le composé du calcium est la chaux vive ou la chaux éteinte.

7. Procédé selon la revendication 4, dans lequel l'additif alcalin est l'hydroxyde de sodium.

8. Produit qui contient les particules agglomérées de phosphate dicalcique anhydre selon la revendication 1, 2 ou 3, lequel produit est un véhicule, une poudre, une capsule ou un agent de nucléation à usage pharmaceutique, ou un véhicule pour additifs alimentaires.

9. Produit selon la revendication 8, qui est un véhicule pharmaceutique.

10. Produit selon la revendication 8, qui est une poudre pharmaceutique.

11. Produit selon la revendication 8, qui est une capsule pharmaceutique.

12. Produit selon la revendication 8, qui est un agent de nucléation pharmaceutique.

13. Produit selon la revendication 8, qui est un véhicule pour additifs alimentaires.
